Europäisches Patentamt

European Patent Office

Office européen des brevets

(11) Publication number: 0 357 337
A2

(12) **EUROPEAN PATENT APPLICATION**

(21) Application number: 89308578.7

(22) Date of filing: 24.08.89

(51) Int. Cl.⁵: **G 01 N 24/00**
// G01N33/48, C12Q1/00

(30) Priority: 27.08.88 GB 8820424

(43) Date of publication of application:
07.03.90 Bulletin 90/10

(84) Designated Contracting States:
AT BE CH DE ES FR GB GR IT LI LU NL SE

(71) Applicant: **FOOD AND VETERINARY LABORATORY LIMITED**
25-26 Frederick Sanger Road The Surrey Research Park
Guildford Surrey (GB)

(72) Inventor: **Parke, Dennis Vernon William "Trevelen"**
Poyle Road
Guildford Surrey (GB)

**Lewis, David Francis Victor**
6 Rupert Road
Guildford Surrey (GB)

(74) Representative: **Heath, Peter William Murray**
FRY HEATH & CO. St. Georges House 6 Yattendon Road
Horley Surrey RH6 7BS (GB)

(54) Methods of evaluation of the toxicity of materials.

(57) The invention provides a method of evaluating toxicity of a test molecular material comprising the steps of preparing from a collection of known molecular materials of known toxicity data relating to a first parameter of shape of the molecule of each of the materials, and data relating to a second parameter of electronic structure of the molecule of each of the materials; determining data concerning the first parameter and the second parameter of the test material for toxicity and comparing the data with respect to its first and second parameters with the data with respect to the first and second parameters of the known materials whereby the anticipated toxicity of the material to be evaluated can be ascertained.

Cytochromes P-450 & P-440
Electronic/Structural Data

Description

## Methods of Evaluation of the Toxicity of Materials

This invention relates to methods for the evaluation of toxicity of chemical compound materials, and more particularly, although not exclusively, relates to the evaluation of carcinogenicity of organic chemical compounds.

It is to be understood that all substances are toxic to a greater or lesser extent, and that environmental exposure doses vary so that the effect varies. In practice, the important criteria in determining acceptable toxicity is whether there is a manifestation of a toxic effect in a test species under a certain specified dose regimen. For humans, the requirement will be the appearance of a toxic response under normal environmental exposure levels, where "environment" can mean ingestion, topical application, or inhalation depending upon the nature of the chemical and on the area of its use.

One of the inherent delays in the development of new chemicals for human use in particular, is the necessity for evaluating the potential toxicity of such chemicals, particularly their carcinogenicity, to ensure that no chemicals of significant carcinogenic potency are released for use.

Traditionally, such testing involves elaborate procedures, particularly on test animals and requires at least two years for completion.

Bearing in mind that such testing has to be carried out with respect to all potential usable chemicals, as well as other compound materials used in association with humans, either for consumption or for handling purposes, and that some have manifestly high carcinogenic potential, whilst other chemicals are of marginal or insignificant carcinogenic toxicity, the lack of any feasible and realistic broad filter to reduce the number of compound substances requiring such expensive and time consuming tests is very keenly felt by those in industries concerned with such matters.

It is an object of the present invention to enable the provision of a broad filter of the kind mentioned above.

In accordance with one aspect of the present invention there is provided a method of evaluating toxicity of a test molecular material comprising the steps of preparing from a collection of known molecular materials of known toxicity data relating to a first parameter of shape of the molecule of each of the materials, and data relating to a second parameter of electronic structure of the molecule of each of the materials; determining data concerning the first parameter and the second parameter of the test material for toxicity and comparing the data with respect to its first and second parameters with the data with respect to the first and second parameters of the known materials whereby the anticipated toxicity of the material to be evaluated can be ascertained.

The first parameter of known materials and the test material may comprise the area divided by the depth squared of the molecule of each of the materials, and the second parameter of the known materials and the test material may comprise the difference in energy between the lowest empty and highest occupied molecular orbitals of the molecule of each of the materials.

The data of the known materials and the data of the test material may be presented pictorially for comparison and evaluation purposes.

The data of the known materials and the data of the test material may be presented in tabular form, or alternatively, may be presented graphically, the first and second parameters comprising cartesion co-ordinates thereof.

Where a graphical representation is used, a band may be marked on the graphical representation separating high toxicity from low toxicity as determined by the positions on the graphical representation of the parameters of the known materials.

Data concerning a third parameter of shape of the known materials and the test material may be collected and included in the comparison and evaluation processes with respect to the test material. The third parameter may be that of length divided by width of the molecule with respect to the known materials and the test material. Where a graphical representation is used, this third parameter may be included as a third cartesion co-ordinate at right angles to the first two thereby providing a three-dimensional graphical representation within the volume of which are plotted the positions of the known materials and the test material whereby to enable comparison and evaluation of that material.

It has been found that the use of a third co-ordinate produces an enhanced separation between chemicals under investigation thereby rendering more effective and useful the comparison and evaluation technique of the invention.

The collection of known molecular materials may comprise materials whose carcinogenic toxicity has been determined previously, at least partly, from their chemical reactions with enzymes or biological receptor systems.

In one embodiment such systems may comprise, for example, the cholinesterases which are involved in the toxicity of pesticides.

In an alternative preferred embodiment of the invention, the collection of known molecular materials comprises materials whose carcinogenic toxicity has been determined previously, at least partly, from induction/inhibition of cytochromes of the family P-450 and of the family P450I by enzyme assays utilising 7-ethoxyresorufin 0-de-ethylase.

The method of the present invention is based upon the fact that the carcinogenic toxicity of a significant number of compounds is known from the induction/inhibition of cytochromes P-450 and P-448 by enzyme assays utilising ethoxyresorufin O-de-ethylase (EROD) (see M D Barker and R T Meyer - "Drug Metabolism and Disposition", 32, 583 (1974)), in combination with other known short term toxicity/mutagenicity tests.

The cytochromes P-450 represent a major class of enzymes which are now known to be responsible for the oxidative metabolism of about 95 per cent of all known chemicals. As well as xenobiotic metabolism, the cytochromes F-450 have an endogenous role in steroid regulation and lipid metabolism. the P-450 isozymes are present mainly in mammalian liver, but are also found in all other species such as birds, plants, fish and bacteria. It has been established that there is a large number of P-450 proteins of which about thirty different forms have been isolated to date, comprising a super-family of isozymes of several phylogeniltically distinct families, which differ significantly in their amino-acid sequences, active sites and substrate specificity. A major family, originally referred to as cytochrome P-448 but now known as P450I, differ markedly from the other P 450 families as they are responsible for the activation of essentially planar substrates to potentially toxic and carcinogenic entities; indeed, many of these planar substrates are also strong inducers of this enzyme family. (See Ioannides et al, Xerobitia, 14 119-137, 1984 and Parke et al, Human Toxicology, 7, 397-404, 1988).

It has been appreciated that, by means of graphical representation, particularly computer graphics, and quantum-chemical calculations, parameters can be defined with respect to the molecular shape and electronic structure of the chemical concerned which can assist in an assessment of the carcinogenic toxic potency of the chemical.

It has been found that, with chemicals of known carcinogenic toxicity, an overwhelming percentage could be separated into those with significant toxicity and those with marginal or insignificant toxicity, with a recognisable intermediate band of doubtful toxicity by means of a graphical representation of the molecular parameters hereinabove defined.

Computer software is available to enable the structure of the molecule of the substance to be evaluated to be produced at a molecular graphics work station coupled to a host computer. In known way, the molecular structure can be minimised to find its lowest energy conformation, and appropriate views selected to enable the molecular dimensions of length, width and depth to be determined. From these criteria, the shape parameter of area divided by depth squared can be calculated. This is a dimensionless ratio.

Known molecular orbital procedures can then be utilised for the calculation of electronic structures. The relevant calculation can be done by means of the host computer of the graphics work station, and from the output data, the difference in energy between the lowest empty (LEMO) and the highest occupied (HOMO) molecular orbitals calculated. For information on molecular orbitals, reference can be made to W G Richards, Quantum Pharmacology, 2nd edition, Butterworths (London) 1983.

Using these parameters as cartesian co-ordinates, the position on a graphical representation gives an assessment of the toxic potential of the substance concerned when compared to the relative positions of chemicals of known toxicity, and in particular in comparison with the transition band between toxicity and non toxicity.

The toxicity evaluation provided by this graphical means provides a broad primary screening test which can be utilised in conjunction with the results of short-term toxicity test procedures to produce a complete carcinogenic toxicity profile of the compound evaluated.

We have found that with present day computers and programs, the assessment according to the invention can take between one and twelve hours per compound for evaluation, whilst a detailed quantitative structure-activity relationship (QSAR) analysis of a series of compounds of similar structure could be undertaken within a few weeks.

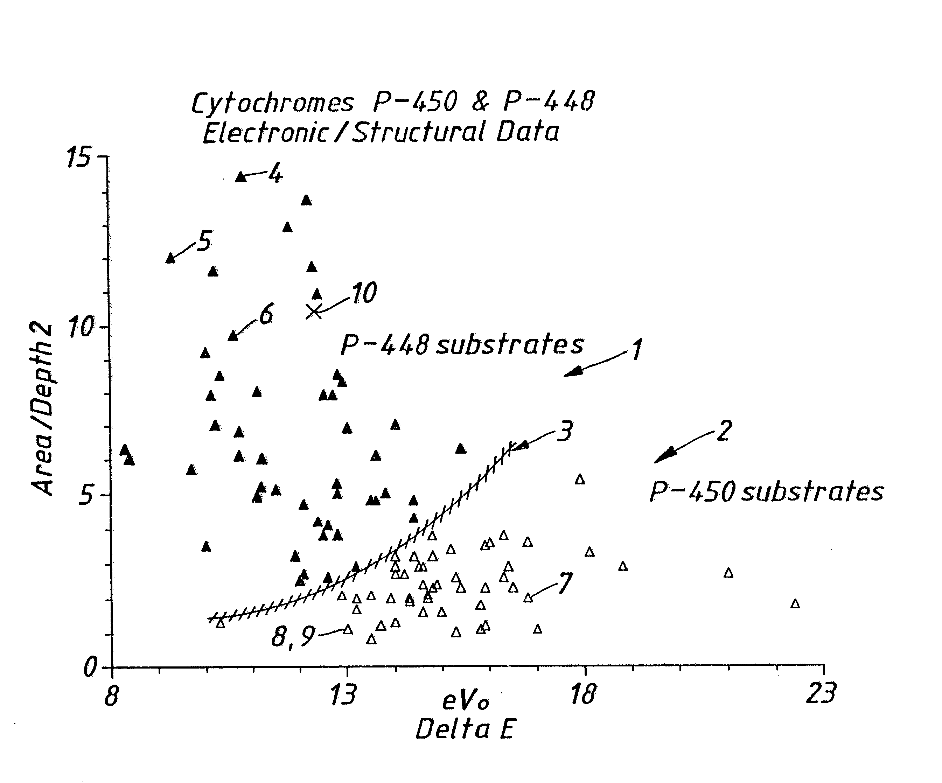

Reference is now made to the drawing which comprises a graph for a number of compound substances of known carcinogenic toxicity with the cartesian co-ordinates of area divided by depth squared against $\triangle E$ (the difference in energy between LEMO and HOMO) for each of the substances. As can be seen, the area 1 of the graph indicates substances having a significant toxicity potential in a recognisable pattern array. The potential toxicity of these substances is known from the EROD enzyme assay showing the induction of cytochromes P-448. Area 2 of the graph shows a pattern grouping of substances of marginal or insignificant toxicity set out in a recognisable pattern substances in this group are of known low toxicity by virtue of the EROD enzyme assay involving the non-induction of cytochromes P-448.

It can generally be stated that areas 1 and 2 can be referred to as P-448 substrates and P-450 substrates respectively.

It is to be seen that the zones 1 and 2 are separated by a band 3 which is to be regarded as the band of indeterminate toxicity or the area of uncertainty.

The assessment of toxicity potency of a molecular substance having undergone determination of its values for the two cartesian co-ordinates is on the basis of pattern recognition tests and essentially determines whether the chemical concerned is likely to be metabolised via cytochromes P-450 or P-448 enzymes which have, as is known, been shown to be intimately linked with the production of toxic metabolites, including carcinogens.

By way of example with reference to the graph Points 4, 5, 6, 7, 8 and 9 represent respectively the following four molecular chemical substances.

| Chemicals | Area/ Depth$^2$ | $\triangle E$ | Specificity |
|---|---|---|---|
| Dibenz(a.h)anthracene | 14.2 | 10.8 | P-448 |
| Benzo(a)pyrene | 12.0 | 9.3 | P-448 |
| N.N-Dimethylamino-azobenzene | 9.7 | 10.6 | P-448 |
| Acetone | 2.0 | 16.8 | P-450 |
| Phenobarbital | 1.1 | 13.0 | P-450 |
| Aldrin | 1.1 | 13.0 | P-450 |

By way of a validation example with respect to the graph, we would refer to 2-amino anthraquinone which has been found by means of the known Ames test and by means of the known Rodent two species assay to have significant carcinogenic toxicity as an area divided by depth squared of 10.3 and a △E of 12.3 and is thus located at Point 10 showing significant toxicity in accordance with the graph.

For further reference confirming such cytochromes see D F V Lewis, Drug Metabolism Reviews, 17, 1 (1986) and C Ioannides & D V Parke, Biochemical Pharmacology, 36, 4197 (1987).

It will be appreciated that determination on the basis of the graphical representation is a relatively straightforward matter, providing a positive prediction in all cases except where the substance to be evaluated falls within the area of uncertainty.

By means of the invention, we have provided a method of evaluating the carcinogenic toxicity potential of compounds, so that, amongst other things, a practical filter is provided with respect to decision making on substances requiring long term testing for their carcinogenic toxicity.

## Claims

1. A method of evaluating toxicity of a test molecular material comprising the steps of preparing from a collection of known molecular materials of known toxicity data relating to a first parameter of shape of the molecule of each of the materials, and data relating to a second parameter of electronic structure of the molecule of each of the materials; determining data concerning the first parameter and the second parameter of the test material for toxicity and comparing the data with respect to its first and second parameters with the data with respect to the first and second parameters of the known materials whereby the anticipated toxicity of the material to be evaluated can be ascertained.

2. A method as claimed in Claim 1 wherein the first parameter of the known materials and the test material comprises the area divided by the depth squared of the molecule of each of the materials and the second parameter of the known materials and the test material comprises the difference in energy between the lowest empty and highest occupied molecular orbital of the molecule of each of the materials.

3. A method as claimed in Claim 1 or 2 wherein the data of the known materials and the data of the test material are presented pictorially for comparison and evaluation purposes.

4. A method as claimed in Claim 3 wherein the data of the known materials and the data of the test material are presented in tabular form.

5. A method as claimed in Claim 3 wherein the first and second parameters are presented graphically, the first and second parameters comprising cartesion co-ordinates thereof.

6. A method as claimed in Claim 5 including the step of marking on the graphical representation of the first and second parameters a band separating high toxicity from low toxicity as determined by the positions on the graphical representation of the parameters of the known materials.

7. A method as claimed in any one of the preceding claims wherein data concerning a third parameter of shape of the known materials and the test material is collected and included in the comparison and evaluation process with respect to the test material.

8. A method as claimed in Claim 7 and Claim 2 wherein the third parameter is that of length divided by width of the molecule of the known materials and the test material.

9. A method as claimed in Claim 8 and Claim 5 wherein the graphical representation includes the third parameter as a third cartesian co-ordinate at right angles to the first two providing a three-dimensional graphical representation within the volume of which are plotted the positions of the known materials and the test material whereby to enable comparison and evaluation of that material.

10. A method as claimed in any one of the preceding claims wherein the collection of known molecular materials comprises materials whose carcinogenic toxicity has been determined previously, at least partly, from the chemical reactions with enzyme or biological receptor systems.

11. A method as claimed in Claim 10 wherein the collection of known molecular materials comprises materials whose carcinogenic toxicity has been determined previously, at least partly, from induction/inhibition of cytochromes of the family P-450 and of the family of P450I by enzyme assays utilising 7-ethoxyresorufin 0-de-ethylase.

12. A method as claimed in any one of the preceding claims wherein the shape and electronic structure parameters of the molecules of the test material are determined utilising computer graphics and quantum-chemical calculations.

Cytochromes P-450 & P-448
Electronic / Structural Data

EP 0 357 337 A2